(19) Europäisches Patentamt
European Patent Office
Office européen des brevets

(11) Veröffentlichungsnummer: **0 112 532**
**A2**

(12) # EUROPÄISCHE PATENTANMELDUNG

(21) Anmeldenummer: **83112469.8**

(22) Anmeldetag: **12.12.83**

(51) Int. Cl.³: **C 07 C 69/712**, C 07 D 213/55, C 07 C 67/14, C 07 C 67/31, A 01 N 43/40, A 01 N 37/02, C 07 C 143/68, C 07 C 43/295, C 07 C 149/20, C 07 C 41/16, C 07 C 69/63

(30) Priorität: **24.12.82 DE 3247929**

(43) Veröffentlichungstag der Anmeldung: **04.07.84 Patentblatt 84/27**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Moriya, Koichi, Namiki-cho 39-15, Hachioji-shi Tokyo (JP)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**

(54) **Phenoxypropionsäure-Derivate.**

(57) Neue Phenoxypropionsäure-Derivate der Formel

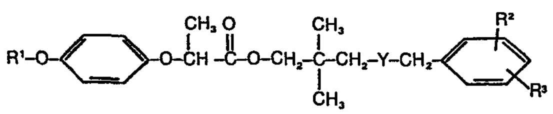

(I)

in welcher

R¹ für die Reste der Formeln

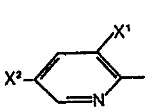 oder 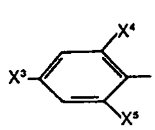 steht,

worin

$X^1$ für Wasserstoff oder Halogen steht, $X^2$ für Halogen oder Trifluormethyl steht, $X^3$ für Halogen oder Trifluormethyl steht, $X^4$ für Wasserstoff oder Halogen steht und $X^5$ für Wasserstoff oder Halogen steht, Y für Sauerstoff oder den Rest $SO_m$ steht, wobei m für 0, 1 oder 2 steht, und $R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

mehrere Verfahren zur Herstellung der neuen Stoffe und deren Verwendung als Herbizide.

Neue Zwischenprodukte sowie Verfahren zu deren Herstellung.

EP 0 112 532 A2

ACTORUM AG

BAYER AKTIENGESELLSCHAFT
Zentralbereich
Patente, Marken und Lizenzen

5090 Leverkusen, Bayerwerk
Dü/bo/c
Ib

## Phenoxypropionsäure-Derivate

Die Erfindung betrifft neue Phenoxypropionsäure-Derivate, mehrere Verfahren zu deren Herstellung und deren Verwendung als Herbizide. Außerdem betrifft die Erfindung neue Zwischenprodukte und ein Verfahren zu deren Herstellung.

Es ist bereits bekannt, daß zahlreiche Phenoxypropionsäure-Derivate herbizide Eigenschaften besitzen (vgl. DE-OS 26 17 804 und US-PS 4 046 553). So können zum Beispiel 2-[4-(3,5-Dichlor-pyridyl-2-oxy)-phenoxy]-propionsäure-benzylester und 2[4-(4-Trifluormethyl-phenoxy)-phenoxy]-propionsäure-(2-phenoxy)-ethylester zur Bekämpfung von Unkraut eingesetzt werden. Die Wirkung dieser Stoffe ist jedoch nicht immer ausreichend.

Es wurden jetzt die neuen Phenoxypropionsäure-Derivate der Formel

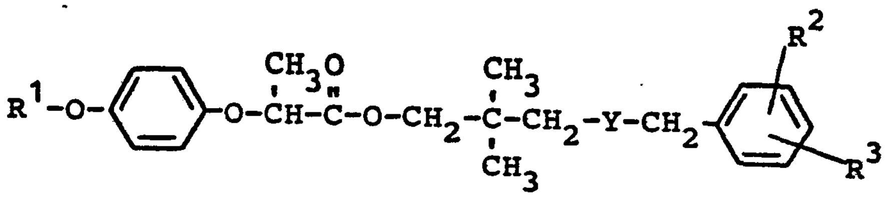

(I)

in welcher

$R^1$ für die Reste der Formeln

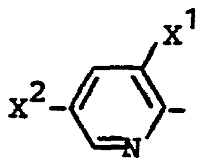 oder 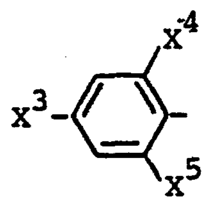 steht,

worin

$X^1$ für Wasserstoff oder Halogen steht,
$X^2$ für Halogen oder Trifluormethyl steht,
$X^3$ für Halogen oder Trifluormethyl steht,
$X^4$ für Wasserstoff oder Halogen steht und
$X^5$ für Wasserstoff oder Halogen steht,

Y für Sauerstoff oder den Rest $SO_m$ steht,

wobei

m für 0, 1 oder 2 steht,

und

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

gefunden.

Die Phenoxypropionsäure-Derivate der Formel (I) enthalten ein asymmetrisches Kohlenstoffatomen im Propionsäureteil und können deshalb in zwei enantiomeren Formen vorliegen. Die Erfindung betrifft sowohl die jeweiligen Racemate als auch die R- und S-Enantiomeren.

Weiterhin wurde gefunden, daß man die neuen Phenoxypropionsäure-Derivate der Formel (I) erhält, wenn man

a) Phenoxypropionsäurechloride der Formel

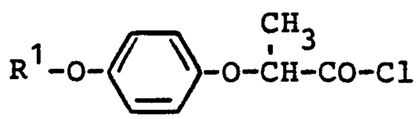

(II)

in welcher

$R^1$ die oben angegebene Bedeutung hat,

mit Hydroxyverbindungen der Formel

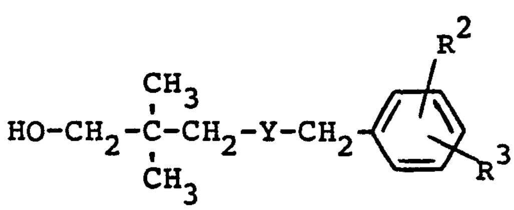

(III)

in welcher

$R^2$, $R^3$ und Y die oben angegebene Bedeutung haben,

gegebenenfalls in Gegenwart eines Säureakzeptors sowie gegebenenfalls in Gegenwart eines Verdünnungsmittels umsetzt und gegebenenfalls anschließend oxidiert,

oder

b) Phenol-Derivate der Formel

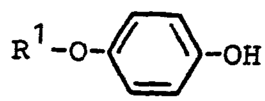

(IV)

in welcher

$R^1$ die oben angegebene Bedeutung hat,

mit Propionsäure-Derivaten der Formel

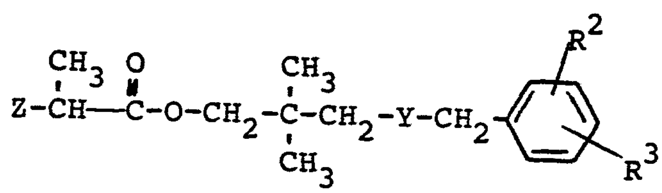

(V)

in welcher

$R^2$, $R^3$ und Y die oben angegebene Bedeutung haben, und

Z für Halogen, Tosylat oder Mesylat steht,

gegebenenfalls in Gegenwart eines Säureakzeptors sowie gegebenenfalls in Gegenwart eines Verdünnungsmittels umsetzt und gegebenenfalls anschließend oxidiert.

Schließlich wurde gefunden, daß sich die neuen Phenoxypropionsäure-Derivate der Formel (I) durch eine hervorragende herbizide Wirksamkeit auszeichnen.

Überraschenderweise besitzen die erfindungsgemäßen Phenoxypropionsäure-Derivate der Formel (I) wesentlich bessere herbizide Eigenschaften als die aus dem Stand der Technik bekannten Verbindungen 2-[4-(3,5-Dichlorpyridyl-2-oxy)-phenoxy]-propionsäure-benzylester und 2-[4-(4-Trifluormethyl-phenoxy)-phenoxy]-propionsäure-(2-phenoxy)-ethylester, welches konstitutionell ähnliche Stoffe analoger Wirkungsart sind.

Die erfindungsgemäßen Stoffe sind durch die Formel (I) allgemein definiert. In dieser Formel steht $R^1$ für die Reste der Formeln

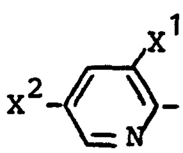 und 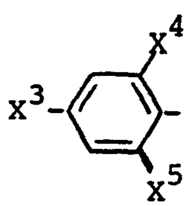 , in welchen

vorzugsweise

$X^1$ für Wasserstoff oder Chlor steht,
$X^2$ für Chlor oder Trifluormethyl steht,
$X^3$ für Chlor oder Trifluormethyl steht,
$X^4$ für Wasserstoff oder Chlor steht und

$X^5$ für Wasserstoff oder Chlor steht.

Y steht für Sauerstoff oder den Rest $SO_m$, wobei m für 0, 1 oder 2 steht, und die Reste $R^2$ und $R^3$ stehen unabhängig voneinander vorzugsweise für Wasserstoff, Fluor, Chlor, Brom, Jod, Alkyl mit 1 oder 2 Kohlenstoffatomen, Alkylthio mit 1 oder 2 Kohlenstoffatomen, Nitro, Cyano oder Alkoxycarbonyl mit 1 oder 2 Kohlenstoffatomen in der Alkoxygruppe.

Besonders bevorzugt sind diejenigen Phenoxypropionsäure-Derivate der Formel (I), in denen $R^1$ für die Reste der Formeln

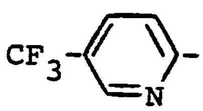 , 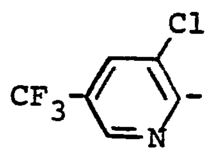 , 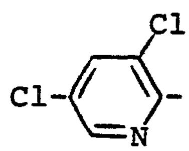 , 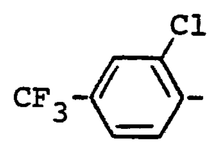 ,

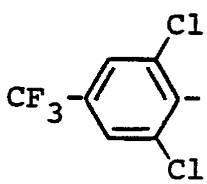 oder 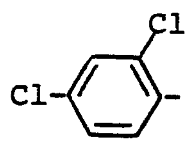 steht und

Y sowie $R^2$ und $R^3$ die oben angegebenen Bedeutungen haben.

Besonders bevorzugt sind auch die R-Enantiomeren dieser besonders bevorzugten Phenoxypropionsäure-Derivate, also diejenigen Verbindungen der Formel

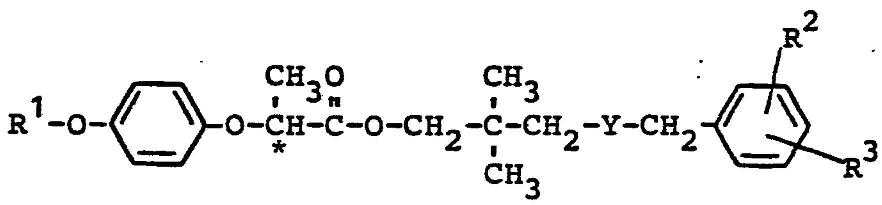 (I),

welche am asymmetrisch substituierten Kohlenstoffatom die R-Konfiguration aufweisen.

In der obigen Formel ist das asymmetrisch substituierte Kohlenstoffatom durch ein (*) gekennzeichnet.

Verwendet man 2-/4-(5-Trifluormethyl-pyridyl-2-oxy)-phenoxy7-propionsäurechlorid und 2,2-Dimethyl-propan-1,3-diol-mono-benzylether als Ausgangsstoffe, so kann der Verlauf des erfindungsgemäßen Verfahrens (a) durch das folgende Formelschema wiedergegeben werden:

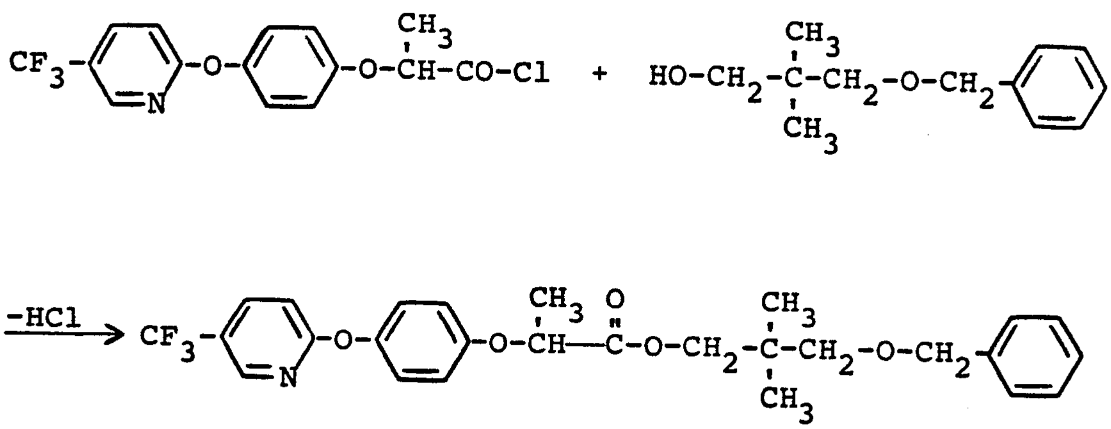


Verwendet man 4-(2-Chlor-4-trifluormethyl-phenoxy)-phenol und 2-Brom-2-propionsäure-(2,2-dimethyl-3-benzyloxy)-propylester als Ausgangsstoffe, so kann der Verlauf des erfindungsgemäßen Verfahrens (b) durch das folgende Formelschema wiedergegeben werden:

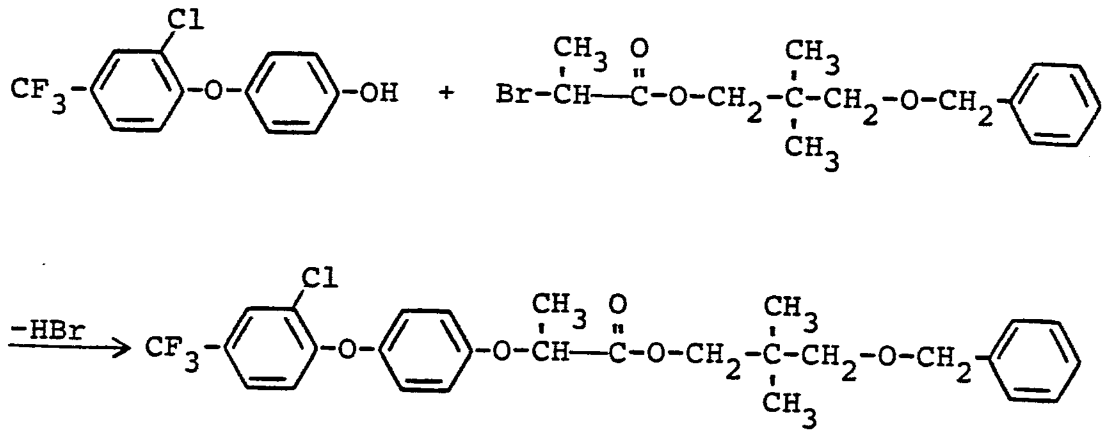


Die bei dem erfindungsgemäßen Verfahren (a) als Ausgangsstoffe benötigten Phenoxypropionsäurechloride sind durch die Formel (II) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für den Substituenten $R^1$ genannt wurden.

Die Phenoxypropionsäurechloride der Formel (II) sind bekannt oder lassen sich nach bekannten Methoden in einfacher Weise herstellen (vgl. Deutsche Offenlegungsschriften 2 628 384, 2 758 002 und 2 812 571 sowie EP-OS 483). So kann man die Stoffe der Formel (II) zum Beispiel dadurch erhalten, daß man die zugrundeliegenden Säuren mit Thionylchlorid umsetzt.

Zur Synthese der R-Enantiomeren der Phenoxypropionsäure-Derivate der Formel (I) werden die R-Enantiomeren der Phenoxypropionsäurechloride der Formel (II) bei der Durchführung des erfindungsgemäßen Verfahrens (a) als Ausgangssubstanzen benötigt. Auch diese

Stoffe sind bekannt oder lassen sich nach bekannten Methoden in einfacher Weise herstellen (vgl. DE-OS 27 58 002).

Die bei dem erfindungsgemäßen Verfahren (a) weiterhin als Ausgangsstoffe benötigten Hydroxyverbindungen sind durch die Formel (III) allgemein definiert. In dieser Formel haben $R^2$, $R^3$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Hydroxyverbindungen der Formel (III) sind bisher noch nicht bekannt. Sie lassen sich dadurch herstellen, daß man Verbindungen der Formel

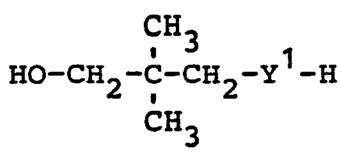

(VI)

in welcher

$Y^1$ für Sauerstoff oder Schwefel steht,

mit Benzylhalogeniden der Formel

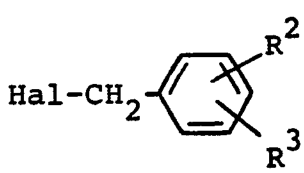

(VII)

in welcher

$R^2$ und $R^3$ die oben angegebene Bedeutung haben und

Hal für Chlor, Brom oder Jod steht,

in Gegenwart eines Säureakzeptors sowie gegebenenfalls in Gegenwart eines Verdünnungsmittels umsetzt und, - sofern $Y^1$ für Schwefel steht - , die dabei entstehenden Stoffe der Formel

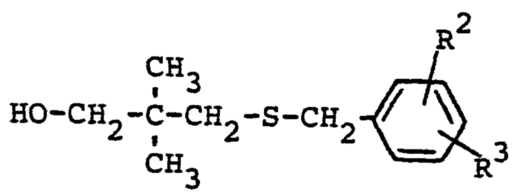

(IIIa)

in welcher

$R^2$ und $R^3$ die oben angegebene Bedeutung haben,

gegebenenfalls mit Wasserstoffperoxid in Gegenwart eines Verdünnungsmittels oxidiert.

Bei dieser Herstellung von Hydroxyverbindungen der Formel (III) können bei der Durchführung der ersten Stufe vorzugsweise alle diejenigen Säureakzeptoren und Verdünnungsmittel eingesetzt werden, die im Zusammenhang mit der Beschreibung des erfindungsgemäßen Verfahrens (a) als bevorzugt genannt werden (vgl. unten).

Bei der Durchführung der Oxidation in der zweiten Stufe des Verfahrens zur Herstellung der Verbindungen der Formel (III) können alle üblicherweise für derartige Reaktionen verwendbaren inerten Lösungsmittel eingesetzt werden. Vorzugsweise verwendbar ist Methylenchlorid.

Die Reaktionstemperaturen können bei dem Verfahren zur Herstellung der Hydroxyverbindungen der Formel (III) in einem größeren Bereich variiert werden. Im allgemeinen arbeitet man in der ersten Stufe bei Temperaturen zwischen -20°C und +160°C, vorzugsweise zwischen 0°C und +140°C. In der zweiten Stufe arbeitet man im allgemeinen bei Temperaturen zwischen -20°C und +50°C, vorzugsweise zwischen 0°C und 40°C.

Bei der Herstellung der Hydroxyverbindungen der Formel (III) nach dem obigen Verfahren setzt man die Reaktionskomponenten in etwa äquimolaren Mengen ein. Es ist jedoch auch möglich, eine der beiden Komponenten in einem größeren Überschuß zu verwenden. Wenn eine Oxidation des zunächst entstehenden Stoffes der Formel (IIIa) gewünscht wird, setzt man das Oxidationsmittel in der jeweils berechneten Menge ein. Die Aufarbeitung erfolgt jeweils nach üblichen Methoden.

Die bei dem erfindungsgemäßen Verfahren (b) als Ausgangsstoffe benötigten Phenol-Derivate sind durch die Formel (IV) definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Phenol-Derivate der Formel (IV) sind bekannt oder lassen sich nach bekannten Methoden in einfacher Weise

herstellen (vgl. DE-OS 27 58 002, DE-OS 28 12 571, EP-OS 483 und EP-OS 1473).

Die bei dem erfindungsgemäßen Verfahren (b) weiterhin als Ausgangssubstanzen benötigten Propionsäure-Derivate sind durch die Formel (V) allgemein definiert. In dieser Formel haben $R^2$, $R^3$ und Y vorzugsweise diejenigen Bedeutungen, die bereits im Zusammenhang mit der Beschreibung der erfindungsgemäßen Stoffe der Formel (I) vorzugsweise für diese Substituenten genannt wurden. Z steht vorzugsweise für Chlor, Brom, Tosylat (-$OSO_2$-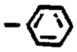-$CH_3$) oder Mesylat (-$OSO_2$-$CH_3$).

Die Propionsäure-Derivate der Formel (V) sind bisher noch nicht bekannt. Sie lassen sich jedoch herstellen, indem man Hydroxyverbindungen der Formel

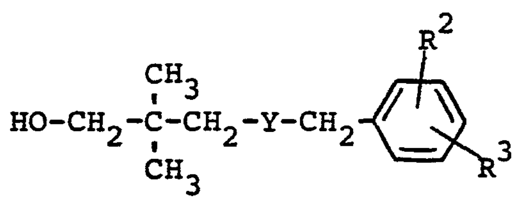

(III)

in welcher

$R^2$, $R^3$ und Y die oben angegebene Bedeutung haben,

mit Milchsäure-Derivaten der Formel

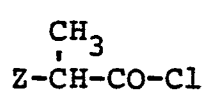

(VIII)

in welcher

Z die oben angegebene Bedeutung hat,

gegebenenfalls in Gegenwart eines Säureakzeptors sowie gegebenenfalls in Gegenwart eines Verdünnungsmittels umsetzt.

Die bei der Herstellung der Propionsäure-Derivate der Formel (V) als Ausgangsstoffe benötigten Hydroxyverbindungen der Formel (III) und deren Herstellung wurden bereits im Zusammenhang mit der Beschreibung des erfindungsgemäßen Verfahrens (a) behandelt. Die für die Umsetzung zur Synthese der Propionsäure-Derivate der Formel (V) außerdem als Reaktionskomponenten benötigten Milchsäure-Derivate der Formel (VIII) sind bekannt oder lassen sich nach bekannten Methoden in einfacher Weise herstellen.

Zur Synthese der R-Enantiomeren der Phenoxypropionsäure-Derivate der Formel (I) nach dem Verfahren (b) ist der Einsatz der S-Enantiomeren der Propionsäure-Derivate der Formel (V) erforderlich, da im Zuge der Umsetzung an dem asymmetrischen Kohlenstoffatom der Propionsäure-Derivate der Formel

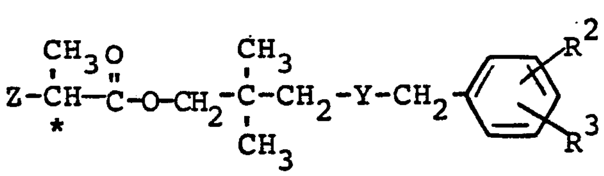

(V)

eine Walden'sche Umkehr erfolgt. In der obigen Formel (V) ist das asymmetrische Kohlenstoffatom durch ein (*)

gekennzeichnet. Die zur Herstellung der S-Enantiomeren der Propionsäure-Derivate der Formel (V) als Ausgangsstoffe benötigten S-Enantiomeren Milchsäure-Derivate der Formel (VIII) sind bekannt oder lassen sich nach bekannten Methoden in einfacher Weise herstellen.

Die erfindungsgemäßen Verfahren (a) und (b) sowie auch das Verfahren zur Herstellung der Stoffe der Formel (V) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone wie Aceton, Methyl-ethyl-, Methylisopropyl-, und Methylisobutyl-keton, Ester, wie Essigsäure-methylester und -ethylester, Nitrile, wie z.B. Acetonitril und Popionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können sowohl bei den erfindungsgemäßen Verfahren (a) und (b) als auch bei dem Verfahren zur Herstellung der Verbindungen der Formel (V) alle üblicherweise für derartige Umsetzungen verwendbaren

Säurebindemittel eingesetzt werden. Vorzugsweise in Frage kommen Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzyl-amin, 1,5-Diazabicyclo/4,3,0/non-5-en (DBN), 1,8-Diazabicyclo/5,4,0/-undec-7-en (DBU) und Pyridin.

Die Reaktionstemperaturen können sowohl bei den erfindungsgemäßen Verfahren (a) und (b) als auch bei dem Verfahren zur Herstellung der Verbindungen der Formel (V) innerhalb eines größeren Bereiches variiert werden. Im allgemeinen arbeitet man jeweils bei Temperaturen zwischen -20°C und +160°C, vorzugsweise zwischen 0°C und +140°C.

Die erfindungsgemäßen Verfahren (a) und (b) und auch das Verfahren zur Herstellung der Verbindungen der Formel (V) werden im allgemeinen unter Normaldruck durchgeführt. Es ist jedoch auch möglich unter erhöhtem oder vermindertem Druck zu arbeiten.

Zur Durchführung der erfindungsgemäßen Verfahren (a) und (b) sowie des Verfahrens zur Herstellung der Verbindungen der Formel (V) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren. Im allgemeinen geht man so vor, daß man das Reaktionsgemisch mit Wasser versetzt, mehrfach mit einem mit Wasser wenig mischbaren organischen Lösungsmittel extrahiert, die vereinigten organischen Phasen trocknet und durch Abziehen des Lösungsmittels einengt.

Die neuen Verbindungen fallen zum Teil in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden können.

Zur Herstellung derjenigen Verbindungen der Formel (I), in denen Y für $SO_m$ steht und m für 1 oder 2 steht, können diejenigen Stoffe der Formel (I), in denen Y für Schwefel steht, nach üblichen Methoden mit der jeweils benötigten Menge oder auch mit einem Überschuß an Oxidationsmittel, gegebenenfalls in Gegenwart eines Katalysators sowie in Gegenwart eines Verdünnungsmittels oxidiert werden.

Als Oxidationsmittel können dabei alle üblichen sauerstoffabgebenden Oxidationsmittel eingesetzt werden. Vorzugsweise in Frage kommen Wasserstoffperoxid, Peressigsäure und m-Chlorperbenzoesäure.

Als Verdünnungsmittel kommen bei der Durchführung dieser Oxidation alle üblicherweise für derartige Oxidationen verwendbaren organischen Lösungsmittel in Betracht. Vorzugsweise verwendbar sind Eisessig und Methylenchlorid.

Als Katalysatoren können bei dieser Oxidation alle üblicherweise für derartige Oxidationen verwendbaren Reaktionsbeschleuniger eingesetzt werden. Vorzugsweise verwendbar sind Ameisensäure und Schwefelsäure.

Die Temperaturen können bei der Oxidation innerhalb eines bestimmten Bereiches variiert werden. Im allgmeinen arbeitet man zwischen -20°C und +50°C, vorzugsweise zwischen 0°C und +40°C.

Bei der Durchführung der Oxidation setzt man die Ausgangsverbindung der Formel (I) im allgemeinen mit der jeweils berechneten Menge oder mit einem geringen Überschuß an Oxidationsmittel um. Die Aufarbeitung erfolgt jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle,

Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

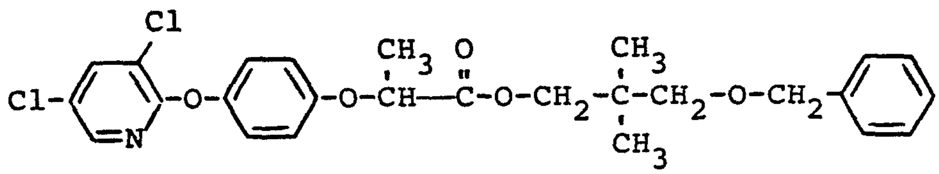

I) (Verfahren b)

Ein Gemisch aus 2,6 g (0,01 Mol) 4-(3,5-Dichlor-pyridyl-2-oxy)-phenol, 3,3 g (0,0097 Mol) 2-Brom-propionsäure-(2,2-dimethyl-3-benzyloxy)-propylester und 1,4 g (0,01 Mol) Kaliumcarbonat in 100 ml Acetonitril wurde 5 Stunden unter Rückfluß erhitzt. Danach wurde das Reaktionsgemisch auf Raumtemperatur abgekühlt und in 200 ml Wasser gegossen. Es wurde zweimal mit je 100 ml Ether extrahiert. Die vereinigten organischen Phasen wurden über Natriumsulfat getrocknet und durch Abziehen des Lösungsmittels unter vermindertem Druck eingeengt. Der verbleibende Rückstand wurde durch leichtes Erwärmen im Hochvakuum von Resten an flüchtigen Anteilen befreit. Man erhielt auf diese Weise 4,5 g (89,3 % der Theorie) an 2-/4-(3,5-Dichlor-pyridyl-2-oxy)-phenoxy/-propionsäure-(2,2-dimethyl-3-benzyloxy)-propylester in Form einer Flüssigkeit vom Brechungsindex $n_D^{20}$ = 1,5542.

Herstellung der Ausgangsprodukte

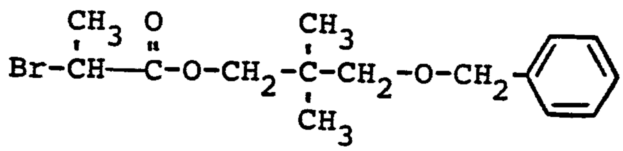

(V-1)

Eine Lösung von 17,2 g (0,1 Mol) 2-Brom-propionylchlorid in 100 ml Toluol wurde bei Raumtemperatur in ein Gemisch aus 194 g (0,1 Mol) (2,2-Dimethyl-3-hydroxy-propyl)-benzylether und 11 g (0,11 Mol) Triethylamin in 200 ml Toluol gegeben. Man rührte weitere 14 Stunden bei Raumtemperatur und arbeitete dann auf, indem man das Reaktionsgemisch zunächst mit Wasser versetzte, dann mehrfach mit Toluol extrahierte, die vereinigten Toluolphasen trocknete und durch Abziehen des Lösungsmittels unter vermindertem Druck einengte. Man erhielt auf diese Weise 19 g (58 % der Theorie) an 2-Brom-propionsäure-(2,2-dimethyl-3-benzyloxy)-propylester.

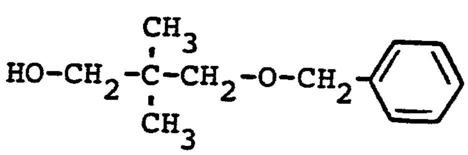

(III-1)

Eine Lösung von 126,5 g (1 Mol) Benzylchlorid in 200 ml Toluol wurde unter Rühren bei 60°C in ein Gemisch aus 208 g (2 Mol) 2,2-Dimethyl-propan-1,3-diol, 56 g (1 Mol) Kaliumhydroxid und 50 ml Wasser in 200 ml Toluol eingetropft. Man rührte weitere 14 Stunden bei 80°C und arbeitete dann auf, indem man das Reaktionsgemisch in Wasser goß, dann mehrfach mit Toluol extrahierte, die vereinigten organischen Phasen trocknete und durch Abziehen des Lösungsmittels unter vermindertem Druck einengte. Der verbleibende Rückstand wurde destilliert. Man erhielt auf diese Weise 100 g (51,5 % der Theorie) an (2,2-Dimethyl-3-hydroxy-propyl)-benzylether.
Siedepunkt: 113°C / 6 mbar

II) Herstellung der Verbindung der Formel

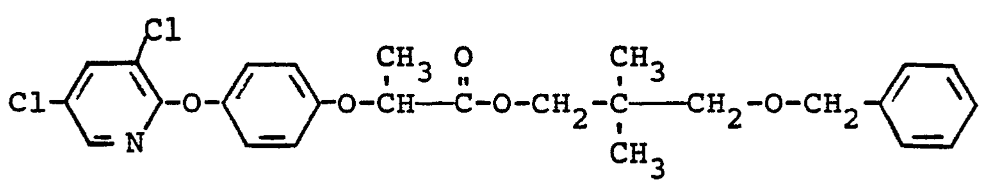

nach der Verfahrensvariante (a):

Zu 3,45 g (0,01 Mol) 2-[4-(3,5-Dichlorpyridyl-2-oxy)-phenoxy]-propionsäurechlorid in 50 ml Toluol tropfte man bei 20°C zunächst 1,9 g (0,01 Mol) (2,2-Dimethyl-3-hydroxy-propyl)-benzylether und dann 1 g (0,01 Mol) Triethylamin.

Man rührte 14 Stunden bei 20°C nach und arbeitete dann das Reaktionsgemisch wie bei der Verfahrensvariante (b) auf.

Man erhielt 4,5 g (89,3 % der Theorie) an 2-[4-(3,5-Dichlor-pyridyl-2-oxy)-phenoxy]-propionsäure-(2,2-dimethyl-3-benzyloxy)-propylester in Form einer Flüssigkeit vom Brechungsindex $n_D^{20}$ = 1,5530.

Nach der im Beispiel 1 unter (II) beschriebenen Methode wurden auch die in den folgenden Beispielen 2 und 3 aufgeführten Verbindungen hergestellt, indem man als Ausgangsstoff das R-Enantiomere des jeweiligen Propionsäurechlorids einsetzte.

Beispiel 2

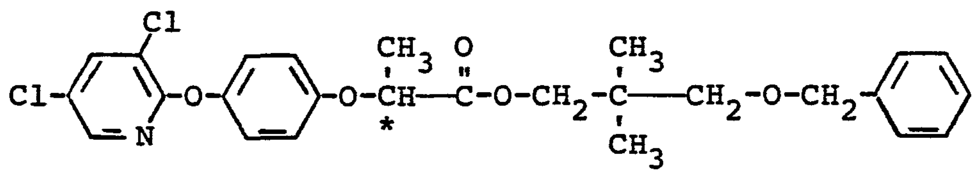

(R)

$$[\alpha]_D^{24} = +3,7^0$$

Beispiel 3

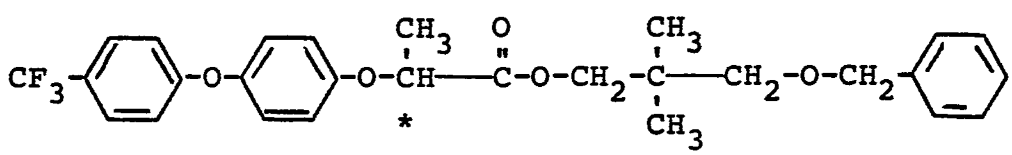

(R)

$$[\alpha]_D^{24} = +3,5^0$$

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Der erfindungsgemäße Wirkstoff (1) zeigt in diesem Test eine sehr gute Wirksamkeit.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Der erfindungsgemäße Wirkstoff (1) zeigt in diesem Test eine sehr gute Wirksamkeit.

## Patentansprüche

1) Phenoxypropionsäure-Derivate der Formel

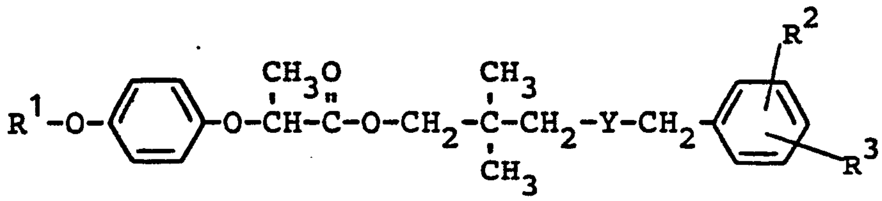 (I)

in welcher

$R^1$ für die Reste der Formeln

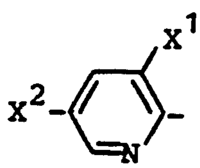 oder 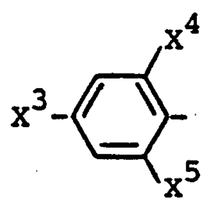 steht,

worin

$X^1$ für Wasserstoff oder Halogen steht,
$X^2$ für Halogen oder Trifluormethyl steht,
$X^3$ für Halogen oder Trifluormethyl steht,
$X^4$ für Wasserstoff oder Halogen steht und
$X^5$ für Wasserstoff oder Halogen steht,

Y für Sauerstoff oder den Rest $SO_m$ steht,

wobei

m für 0, 1 oder 2 steht,

und

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen.

2) Verfahren zur Herstellung von Phenoxypropionsäure-Derivaten der Formel

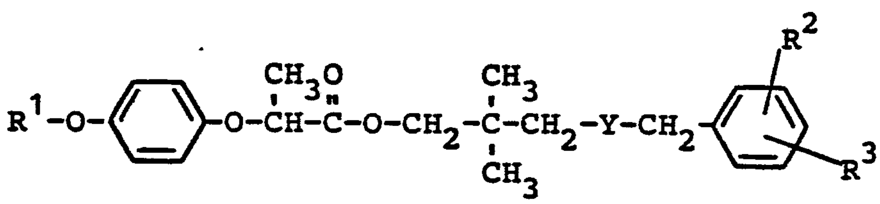

in welcher

$R^1$ für die Reste der Formeln

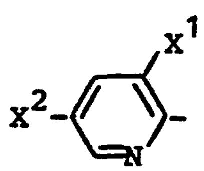

oder

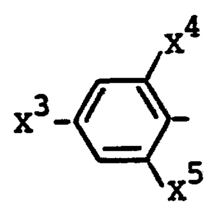

steht,

worin

$X^1$ für Wasserstoff oder Halogen steht,
$X^2$ für Halogen oder Trifluormethyl steht,
$X^3$ für Halogen oder Trifluormethyl steht,
$X^4$ für Wasserstoff oder Halogen steht und
$X^5$ für Wasserstoff oder Halogen steht,

Y für Sauerstoff oder den Rest $SO_m$ steht,

wobei

m für 0, 1 oder 2 steht,

und

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

dadurch gekennzeichnet, daß man

a) Phenoxypropionsäurechloride der Formel

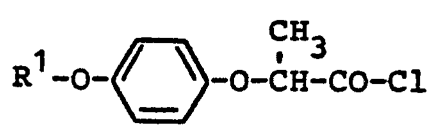

(II)

in welcher

$R^1$ die oben angegebene Bedeutung hat,

mit Hydroxyverbindungen der Formel

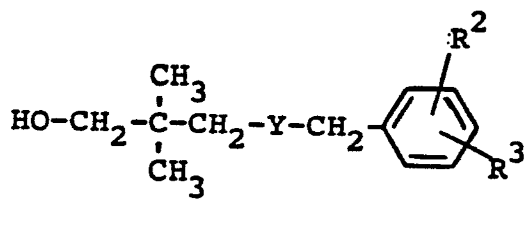

(III)

in welcher

$R^2$, $R^3$ und Y die oben angegebene Bedeutung haben,

gegebenenfalls in Gegenwart eines Säureakzeptors sowie gegebenenfalls in Gegenwart eines Verdünnungsmittels umsetzt und gegebenenfalls anschließend oxidiert,

oder

b) Phenol-Derivate der Formel

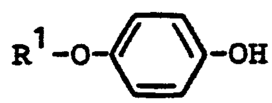 (IV)

in welcher

$R^1$ die oben angegebene Bedeutung hat,

mit Propionsäure-Derivaten der Formel

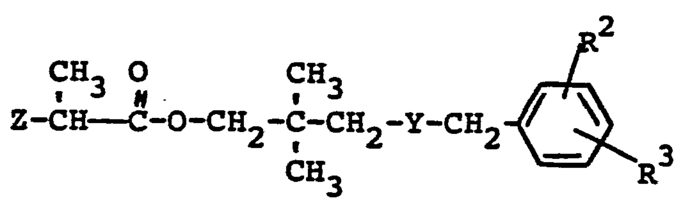 (V)

in welcher

$R^2$, $R^3$ und Y die oben angegebene Bedeutung haben, und

Z    für Halogen, Tosylat oder Mesylat steht,

gegebenenfalls in Gegenwart eines Säureakzeptors sowie gegebenenfalls in Gegenwart eines Verdünnungsmittels umsetzt und gegebenenfalls anschließend oxidiert.

3) Herbizide Mittel, gekennzeichnet durch einen Gehalt an mindestens einem Phenoxypropionsäure-Derivat der Formel (I).

4) Verwendung von Phenoxypropionsäure-Derivaten der Formel (I) zur Bekämpfung von Unkräutern.

5) Verfahren zur Herstellung von herbziden Mitteln, dadurch gekennzeichnet, daß man Phenoxypropionsäure-Derivate der Formel (I) mit Streckmitteln und/oder oberflächenaktiven Stoffen vermischt.

6) Hydroxyverbindungen der Formel

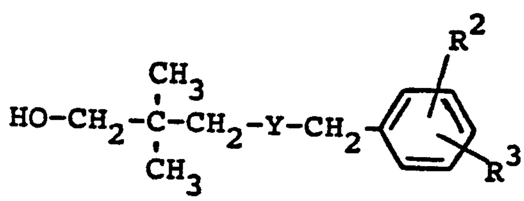

(III)

in welcher

Y für Sauerstoff oder den Rest $SO_m$ steht,

wobei

m für 0, 1 oder 2 steht,

und

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen.

7) Verfahren zur Herstellung von Hydroxyverbindungen der Formel

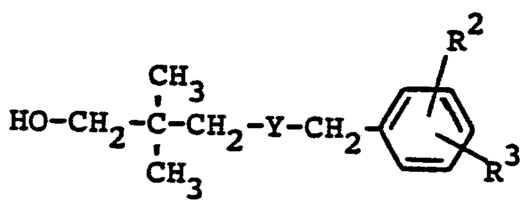

(III)

in welcher

Y für Sauerstoff oder den Rest $SO_m$ steht,

wobei

m für 0, 1 oder 2 steht,

und

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

dadurch gekennzeichnet, daß man Verbindungen der Formel

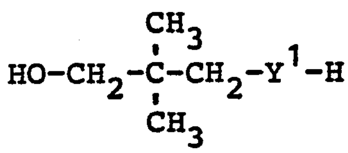

(VI)

in welcher

$Y^1$ für Sauerstoff oder Schwefel steht,

mit Benzylhalogeniden der Formel

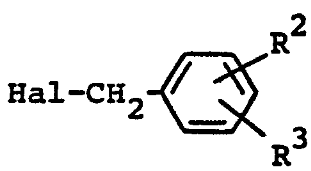

(VII)

in welcher

$R^2$ und $R^3$ die oben angegebene Bedeutung haben und

Hal für Chlor, Brom oder Jod steht,

in Gegenwart eines Säureakzeptors sowie gegebenenfalls in Gegenwart eines Verdünnungsmittels umsetzt

und, - sofern $Y^1$ für Schwefel steht - , die dabei entstehenden Stoffe der Formel

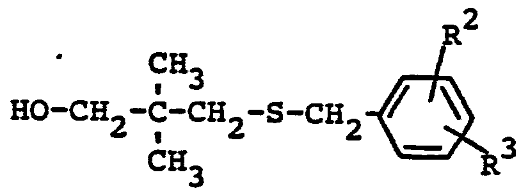

(IIIa)

in welcher

$R^2$ und $R^3$ die oben angegebene Bedeutung haben,

gegebenenfalls mit Wasserstoffperoxid in Gegenwart eines Verdünnungsmittels oxidiert.

8) Propionsäure-Derivate der Formel

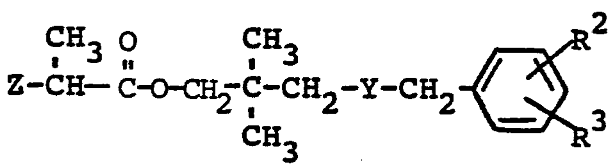

(V)

in welcher

Y für Sauerstoff oder den Rest $SO_m$ steht,

wobei

m für 0, 1 oder 2 steht,

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkyl-

0112532

thio mit 1 bis 4 Kohlenstoffatomen, Nitro, Cyano oder Alkoxycarbonyl mit 1 bis 4 Kohlenstoffatomen in der Alkoxygruppe stehen und

Z für Halogen, Tosylat oder Mesylat steht.

9) Verfahren zur Herstellung von Propionsäure-Derivaten der Formel

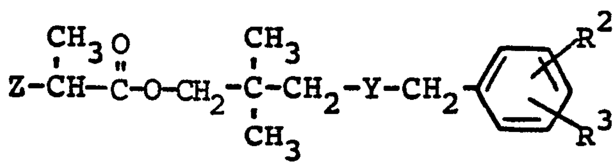

(V)

in welcher

Y für Sauerstoff oder den Rest $SO_m$ steht,

wobei

m für 0, 1 oder 2 steht,

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkylgruppe stehen und

Z für Halogen, Tosylat oder Mesylat steht,

dadurch gekennzeichnet, daß man Hydroxyverbindungen der Formel

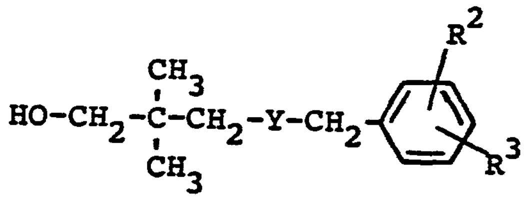

(III)

in welcher

$R^2$, $R^3$ und Y die oben angegebene Bedeutung haben,

mit Milchsäure-Derivaten der Formel

$$Z-\overset{CH_3}{\overset{|}{C}}H-CO-Cl \qquad (VIII)$$

in welcher

Z die oben angegebene Bedeutung hat,

gegebenenfalls in Gegenwart eines Säureakzeptors sowie gegebenenfalls in Gegenwart eines Verdünnungsmittels umsetzt.

10) R-Enantiomere von Phenoxypropionsäure-Derivaten der Formel

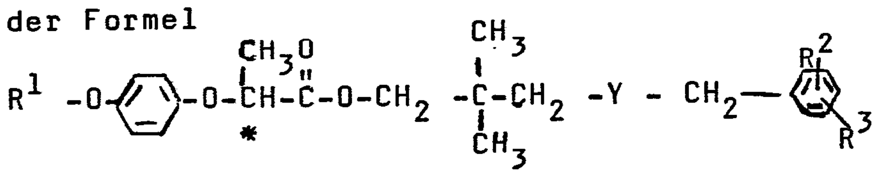

( R )

in welcher

$R^1$ für die Reste der Formeln

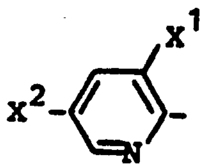 oder 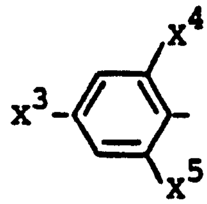 steht,

worin

$X^1$ für Wasserstoff oder Halogen steht,
$X^2$ für Halogen oder Trifluormethyl steht,
$X^3$ für Halogen oder Trifluormethyl steht,
$X^4$ für Wasserstoff oder Halogen steht und
$X^5$ für Wasserstoff oder Halogen steht,

Y für Sauerstoff oder den Rest $SO_m$ steht,

wobei

m für 0, 1 oder 2 steht,

und

$R^2$ und $R^3$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen.